Europäisches Patentamt

European Patent Office

Office européen des brevets

(11) Veröffentlichungsnummer: **0 270 828 B1**

(12) **EUROPÄISCHE PATENTSCHRIFT**

(45) Veröffentlichungstag der Patentschrift: **20.05.92**

(21) Anmeldenummer: **87116092.5**

(22) Anmeldetag: **02.11.87**

(51) Int. Cl.⁵: **A61N 1/08**, H02H 9/00

(54) **Reizstromgerät.**

(30) Priorität: **13.11.86 DE 3638709**

(43) Veröffentlichungstag der Anmeldung:
**15.06.88 Patentblatt 88/24**

(45) Bekanntmachung des Hinweises auf die
Patenterteilung:
**20.05.92 Patentblatt 92/21**

(84) Benannte Vertragsstaaten:
**AT BE CH DE FR LI NL**

(56) Entgegenhaltungen:
**EP-A- 0 173 419**
**EP-A- 0 269 844**
**US-A- 4 088 141**
**US-A- 4 177 819**
**US-A- 4 327 326**

(73) Patentinhaber: **SIEMENS AKTIENGESELL-
SCHAFT
Wittelsbacherplatz 2
W-8000 München 2(DE)**

(72) Erfinder: **Herzog, Ludwig
Drei-Thorn-Strasse 3
W-6948 Waldmichelbach(DE)**
Erfinder: **Knapp, Volker
Pestalozzistrasse 19
W-6948 Waldmichelbach(DE)**

## Beschreibung

Die Erfindung bezieht sich auf ein Reizstromgerät, insbesondere für Reizstromtherapie an einem Patienten, mit einem Reizstromimpulserzeuger und einer Intensitätseinstelleinrichtung mit Intensitätseinstellglied sowie einer Stromabtasteinrichtung für Ist-Werte des Stromes.

Aus der US-A 4 177 819 ist ein Reizstromgerät der eingangs genannten Art bekannt, das eine Sicherheitsschaltung aufweist, die den zu angeschlossenen Elektroden fließenden Strom abtastet und die Reizstromwellen abschaltet, wenn eine No Load oder Overload Bedingung detektiert wird. Das Reizstromgerät enthält einen Leistungsverstärker mit einem Verstärkungssteller, der mit einem Schalter derart verbunden ist, daß vor dem Beginn jeder Behandlung der Verstärkungssteller zur Erzeugung der Reizstromwellen auf Null gestellt werden muß

Die ältere EP-A 0 269 844 die Stand der Technik gemäß Art. 54(3) EPÜ betrifft offenbart ein Reizstromgerät der eingangs genannten Art, das zur Erzeugung eines Abschaltsignales eine Stromtransformationseinrichtung aufweist, die abgetastete Ist-Werte in Vergleichsstromwerte transformiert, die um einen vorgebbaren Bruchteil niedriger sind als die abgetasteten Ist-Werte. Dabei werden die transformierten Vergleichsstromwerte in einer Vergleicheinrichtung mit einem vorgebbaren Referenzstromwert verglichen, wodurch das Abschaltsignal für einen inneren Stromkreis erzeugt wird, sobald ein transformierter Vergleichsstromwert den Referenzstromwert überschreitet.

Bei Reizstromgeräten dieser Art muß sichergestellt werden, daß der zu behandelnde Patient nicht zu rasch mit zu hohen Intensitätswerten des Reizstromes beaufschlagt wird.

Aufgabe vorliegender Erfindung ist es, bei einem Reizstromgerät der eingangs genannten Art Mittel vorzusehen, die einen zu raschen Stromintensitätsanstieg verhindern.

Die Aufgabe wird erfindungsgemäß durch die Merkmale des Patentanspruches 1 gelöst.

Gemäß der Erfindung wird jetzt im Reizstromgerät ein zu rascher Stromintensitätsanstieg bei Einstellung des Intensitätseinstellgliedes erkannt und daraufhin der innere Stromkreis des Reizstromgerätes abgeschaltet.

In bevorzugter Ausgestaltung der Erfindung wird die Abschaltung des inneren Stromkreises erst dadurch wieder aufgehoben, daß das Intensitätseinstellglied auf Null zurückgestellt und dann die Intensität langsam wieder erhöht wird.

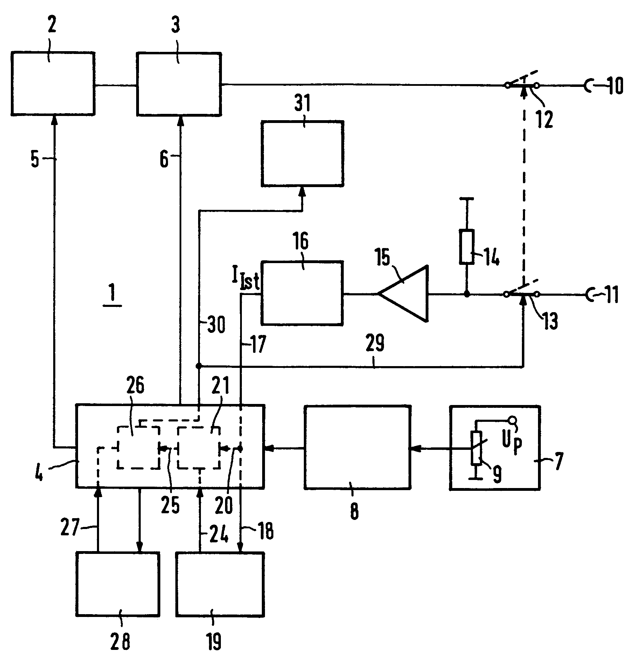

Weitere Vorteile und Einzelheiten der Erfindung ergeben sich aus der nachfolgenden Beschreibung eines Ausführungsbeispiels anhand der Zeichnung und in Verbindung mit den Unteransprüchen.

Die Figur zeigt das erfindungsgemäße Reizstromgerät im Prinzipschaltbild.

Das Reizstromgerät 1 umfaßt unter anderem einen Reizstromimpulserzeuger 2 mit Intensitätseinstelleinrichtung 3, die von einem Mikroprozessor 4 über Leitungen 5, 6 gesteuert werden. Der Intensitätseinstelleinrichtung 3 ist dabei über den Mikroprozessor 4 ein Intensitätseinstellglied 7 mit vorgeschaltetem Analog-Digital-Wandler 8 zugeordnet. Das Intensitätseinstellglied 7 umfaßt als Einstellmittel ein Drehpotentiometer 9 mit Drehknopf (nicht dargestellt). Die Versorgungsspannung des Drehpotentiometers ist mit $U_P$ bezeichnet.

Die Buchsen 10, 11 sind die Geräteanschlußbuchsen für die (nicht dargestellten) Reizstromelektroden. Die (Relais)Schaltkontakte 12, 13 unterbrechen bei Betätigung (gestrichelte Stellung) den inneren Stromkreis des Reizstromgerätes 1. Damit kann bei Unterbrechung kein Reizstrom zum über die Elektroden angeschlossenen Patienten gelangen.

Zur Messung der Istwerte des Stromes beinhaltet das Reizstromgerät 1 ferner einen Meßwiderstand 14, dem über einen Verstärker 15 ein Analog-Digital-Wandler 16 nachgeschaltet ist. Der Analog-Digital-Wandler 16 tastet, gesteuert vom Mikroprozessor 4, den am Widerstand 14 anfallenden stromproportionalen Spannungsabfall ca. 20 mal pro Sekunde ab (Istwerte des Stromes).

Jeder über die Leitung 17 erfaßte Istwert $I_{Ist}$ wird vom Mikroprozessor 4 einerseits über eine Leitung 18 in ein RAM 19 eingespeichert und andererseits über eine Leitung 20 einer Vergleichereinheit 21 des Mikroprozessors 4 zugeleitet. In der Vergleichereinheit 21 vergleicht der Mikroprozessor 4 den jeweils vorhergehend im RAM 19 abgespeicherten Stromwert (abgerufen über Leitung 24) mit dem gerade angefallenen Istwert. Die Differenzgröße zwischen den beiden Stromwerten wird über eine Leitung 25 auf eine weitere Vergleichereinheit 26 des Mikroprozessors gegeben. Dort vergleicht der Mikroprozessor 4 das Differenzsignal mit einem Referenzwert, der vom Mikroprozessor 4 über eine Leitung 27 von einem EPROM 28 abrufbar ist. Übersteigt das Differenzsignal den Referenzwert, so steuert der Mikroprozessor über die Leitung 29 die Kontakte 12, 13 in dem Sinne, daß sie in die gestrichelte Schaltstellung gebracht werden. Der innere Stromkreis des Reizstromgerätes ist damit vom Patienten abgeschaltet. Gleichzeitig aktiviert der Mikroprozessor 4 auch noch über die Leitung 30 eine optische und akustische Alarmanzeige 31.

Das Reizstromgerät reagiert also auf zu rasche Intensitätssteigerung am Intensitätseinstellglied 7 (Drehpotentiometer 9 wurde z.B. zu schnell aus der Nullstellung hochgedreht) durch Abschaltung des inneren Stromkreises und durch entsprechende Alarmanzeige. Eine Wiederanschaltung des inneren Stromkreises (die Kontakte 12, 13 gehen in die durchgezogen dargestellte Schaltstellung) sowie eine Abschaltung des Alarms ist erst wieder dadurch möglich, daß das Drehpotentiometer 9 des Intensitätseinstellgliedes7 in die Nullstellung zurückgedreht und dann anschließend die Intensität langsam wieder erhöht wird.

**Patentansprüche**

1. Reizstromgerät, insbesondere für Reizstromtherapie an einem Patienten, mit einem Reizstromimpulserzeuger und einer Intensitätseinstelleinrichtung mit Intensitätseinstellglied sowie einer Stromabtasteinrichtung für Istwerte des Stromes, wobei der Stromabtasteinrichtung (14 bis 16) eine Vergleichereinheit (21, 26) eines Mikroprozessors (4) nachgeschaltet ist, in der der Mikroprozessor (4) wenigstens bei jeder durch das Intensitätseinstellglied (7) vorgenommenen Intensitätsänderung einen vorhergehend in einem Speicher (19) abgespeicherten Stromwert mit einem gerade angefallenen Stromistwert ($I_{Ist}$) zur Erfassung der Differenz vergleicht und ein Abschaltsignal für den inneren Stromkreis (12, 13) des Gerätes erzeugt, wenn der erfaßte Differenzwert durch eine zu schnelle Betätigung des Intensitätseinstellgliedes (7) einen vorgegebenen Referenzwert überschreitet.

2. Reizstromgerät nach Anspruch 1, **dadurch gekennzeichnet,** daß der Referenzwert in einem EPROM (28) gespeichert ist.

3. Reizstromgerät nach Anspruch 1 oder 2, **dadurch gekennzeichnet,** daß das Abschaltsignal auch noch einen optischen und/oder akustischen Alarmgeber (31) aktiviert.

4. Reizstromgerät nach einem der Ansprüche 1 bis 3, **dadurch gekennzeichnet,** daß eine Wiederanschaltung des inneren Stromkreises sowie im Falle einer Alarmgebung eine Abschaltung des Alarmes erst dann durch den Mikroprozessor (4) erfolgt, wenn das Intensitätseinstellglied (7) auf Null zurückgestellt wird.

**Claims**

1. Stimulus-current device, in particular for stimulus-current therapy on a patient, having a stimulus-current pulse generator and an intensity-setting device with intensity-setting element as well as a current-scanning device for actual values of the current, in which a comparator unit (21, 26) of a microprocessor (4) is connected in series with the current-scanning device (14 to 16), in which comparator unit the microprocessor (4) at least with each intensity change carried out by means of the intensity-setting element (7) compares a current value, previously stored in a memory (19), with an actual current value ($I_{Ist}$) which has just resulted, for the determination of the difference and generates an interrupting signal for the internal circuit (12, 13) of the device, if the determined differential value exceeds a specified reference value through too fast an actuation of the intensity-setting element (7).

2. Stimulus-current device according to claim 1, characterized in that the reference value is stored in an EPROM (28).

3. Stimulus-current device according to claim 1 or 2, characterized in that the interrupting signal also activates an optical and/or acoustic alarm generator (31).

4. Stimulus-current device according to one of claims 1 to 3, characterized in that a reconnection of the internal circuit as well as, in the event of the generation of an alarm, a disconnection of the alarm only takes place by means of the microprocessor (4) if the intensity-setting element (7) is reset to zero.

**Revendications**

1. Stimulateur, notamment pour la thérapie utilisant un courant de stimulation et appliquée à un patient, comportant un générateur d'impulsions de courant de stimulation et un dispositif de réglage de l'intensité comportant un circuit de réglage de l'intensité ainsi qu'un dispositif d'échantillonnage du courant pour l'obtention de valeurs réelles du courant et dans lequel en aval du dispositif d'échantillonnage du courant (14,16) est branchée une unité de comparaison (21,26) d'un microprocesseur (4), et dans lequel le microprocesseur (4) compare, au moins lors de chaque variation de l'intensité exécutée au moyen du circuit (7) de réglage de l'intensité, une valeur du courant mémorisée au préalable dans une mémoire (19) à une valeur réelle du courant ($I_{réel}$), qui existe précisément, pour déterminer la différence et produit un signal d'interruption pour le circuit intérieur (12,13) de l'appareil, lorsque la valeur de différence détectée dépasse une valeur de référence prédéterminée sous l'effet d'un actionnement trop rapide du circuit (7) de réglage de l'intensité.

2. Stimulateur suivant la revendication 1, caractérisé par le fait que la valeur de référence est mémorisée dans une mémoire EPROM (28).

3. Stimulateur suivant la revendication 1 ou 2, caractérisé par le fait que le signal d'interruption active en outre également un générateur d'alarme optique et/ou acoustique (31).

4. Stimulateur suivant l'une des revendications 1 à 3, caractérisé par le fait qu'un nouveau raccordement du circuit intérieur ainsi que, dans le cas de la délivrance d'une alarme, une interruption de l'alarme ne sont exécutés par le microprocesseur (4) que lorsque le circuit (7) de réglage de l'intensité est ramené à zéro.